(19) Europäisches Patentamt
European Patent Office
Office européen des brevets

(11) Publication number: **0 005 280 B1**

# (12) EUROPEAN PATENT SPECIFICATION

(45) Date of publication of patent specification: **13.05.81**

(21) Application number: **79200097.8**

(22) Date of filing: **27.02.79**

(51) Int. Cl.³: **C 07 C 45/00**, C 07 C 47/02, C 07 C 47/20, C 07 C 47/28, C 07 C 47/32, C 07 C 47/45, C 07 C 47/228, C 07 C 47/52, C 07 C 47/54, C 07 C 47/55, C 07 C 47/58 // C01B6/23, C07B1/00

(54) **A process for the reduction of carboxylic acid halides to corresponding aldehydes.**

(30) Priority: **13.03.78 GB 981578**
**12.10.78 GB 4024978**

(43) Date of publication of application: **14.11.79 Bulletin 79/23**

(45) Publication of the grant of the patent: **13.05.81 Bulletin 81/19**

(84) Designated Contracting States: **BE CH DE FR GB IT NL**

(56) References cited:
**US-A-3 147 272**
**US-A-3 277 178**

**ANGEWANDTE CHEMIE, Vol. 65, january 7, 1953, WEINHEIM/BERGSTRASSE**
**E. WIBERG: «Neuere Ergebnisse der pröparativen drid-Forschung», pages 16-33**

(73) Proprietor: **SHELL INTERNATIONALE RESEARCH MAATSCHAPPIJ B.V., Carel van Bylandtlaan 30, NL-2596 HR DEN HAAG (NL)**

(72) Inventor: **Entwistle, Ian David, 44 Woodside Gardens, Sittingbourne Kent (GB)**
Inventor: **Johnstone, Robert Alexander Walker, Dept. of Organic Chemistry University of Liverpool, P.O. Box 147 Liverpool L69 3BX (GB)**

(74) Representative: **Keuzenkamp, Abraham et al, c/o Shell Internationale Research Maatschappij B.V. P.O. Box 302, NL-2501 CH 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

A process for the reduction of carboxylic acid halides to corresponding aldehydes

The invention relates to a process for the reduction of carboxylic acid halides to aldehydes.

A widely-used process for the catalytic reduction of carboxylic acid chlorides to the corresponding aldehydes is the Rosenmund reduction. In this process, carboxylic acid chlorides are hydrogenated using a suitable catalyst, usually 5% palladium on barium sulphate, poisoned with a mixture of quinoline and sulphur to prevent further reduction to the alcohol. This process has the economic disadvantage that pressure equipment is required. An additional disadvantage is that certain functional groups other than the acid chloride group are reduced: for example, nitro groups are reduced to amino groups.

Most hydride reducing agents reduce carboxylic acid halides to the corresponding alcohols. However, at very low temperatures for example -80°C to -70°C, US P 3147272 teaches, inter alia, the use of lithium tri-t-butoxyaluminiumhydride to reduce acid halides to the corresponding aldehydes. Certain complex hydride reducing agents can be used for the reduction of specific acid chlorides to aldehydes; for example, sodium trimethoxyborohydride reduces most acid chlorides to the corresponding alcohol, but reduces triacetylshikimic acid chloride to the corresponding aldehyde.

It is also known from US Patent Specification 3277178 that certain acid halides can be reduced to the corresponding aldehydes by means of alkali metal borohydrides in the presence of a tertiary amine which is at least partially soluble in the reaction medium and which is capable of forming a stable amine borone.

We have now found a method of reducing carboxylic acid halides to give the corresponding aldehyde avoiding the need to use catalytic hydrogenation reactions and the need to use very low temperatures required by known reducing agents.

The present invention provides a process for the reduction of a carboxylic acid halide to the corresponding aldehyde, which comprises reacting the acid halide with cadmium borohydride as reducing agent.

The reducing agent is suitably used in the form of a solution which can be conveniently obtained by mixing a solution or suspension of a cadmium salt with a solution or suspension of sodium borohydride and the term "cadmium borohydride" is employed broadly to denote the presence of cadmium ions and borohydride ions in molar ratios of substantially 1:2. The invention therefore also provides a process for the reduction of a carboxylic acid halide to the corresponding aldehyde, which comprises reacting the acid halide with a solution of cadmium borohydride in an inert polar solvent. Conveniently this solution may be produced in situ.

Preferably the acid halide is an acid bromide or an acid chloride.

The reducing agent is suitably formed by mixing a cadmium salt, conveniently a cadmium halide, with an alkali metal borohydride in an inert polar solvent, and the resulting solution itself is conveniently used directly in the process of the invention.

The alkali metal borohydride may be lithium, sodium or potassium borohydride and is preferably potassium or sodium borohydride. Sodium borohydride is most preferred.

The cadmium halide may be cadmium fluoride, chloride, bromide or iodide and is advantageously cadmium chloride or cadmium bromide. Cadmium chloride is most preferred.

The inert polar solvent should be capable of dissolving at least small quantities of the reactants, although complete solubility of the alkali metal borohydride and the cadmium salt is not required. Alkali metal borohydrides and cadmium halides are relatively insoluble in many common solvents, and therefore the inert polar solvent chosen may comprise a mixture of solvents. Solvent mixtures of acetonitrile with a co-solvent such as diglyme, hexamethylphosphoramide, or, especially dimethylformamide are particularly advantageous. It is not essential to take special steps to dry the solvent, since the preparation of the reducing agent, and the subsequent reaction with a carboxylic acid halide, will proceed in the presence of traces of water.

If the carboxylic acid halide is a liquid, the reduction process of the invention may be accomplished by mixing the carboxylic acid halide directly with the solution of the reducing agent described above. Whether or not the carboxylic acid halide is a liquid, the process of the invention is conveniently effected by mixing the solution of the reducing agent described above with a solution of the carboxylic acid halide in the same solvent as that in the solution of the reducing agent, or, where that solvent is a mixture, in one or more of the solvents present in the mixture.

In preparing the reducing agent an excess of either the alkali metal hydride or the cadmium salt may be used, although it is preferred to use an excess, such as 5 to 10% molar excess, of the cadmium salt rather than an excess of alkali metal hydride, since the presence of excess alkali metal hydride in the process of the invention tends to affect the course of the reduction and tends to favour an increase in formation of alcohol by-product at the expense of the yield of aldehyde.

When performing the reduction, suitably the reducing agent is used in slight molar excess over the carboxylic acid halide, for example 5 to 10% molar excess.

The reduction process of the invention has the advantage that, unlike many hydride reductions, extremely low temperatures are not required. The process of the invention may be performed, for example, at a temperature in the range from

-35 °C to 0 °C, at which temperature satisfactory yields are obtained.

A further advantage of the use of the reduction process of the invention is that it appears to be relatively selective in its action. For example ester groups, cyano groups, nitro groups, halogen atoms and carbon-carbon double bonds are not reduced. Expensive pressure equipment is furthermore not required.

The selectivity of the reduction process to halogen substituents other than that in the carboxy halide group of the carboxylic acid halide is particularly worthy of note. Even relatively reactive halogen, e.g. bromo and chloro, substituents in haloalkyl and arylhaloalkyl groups have been found to be unaffected by the reaction conditions of the process of the invention.

The process of the invention may be used for the reduction of aromatic, cycloaliphatic, or aliphatic acid halides. The reaction may be represented by the equation:

$$R.CO.Hal \xrightarrow{BH_4^-} R.CO.H$$

R may represent, for example, an aryl or aralkyl group, for example a phenyl or benzoyl group; an alkyl, alkenyl or alkynyl group, suitably having up to 10 carbon atoms, for example an octyl group or a 1-propenyl group; or a cycloalkyl group, suitably having from 3 to 6 ring carbon atoms, for example a cyclopropyl or a cyclohexyl group. Any of the above groups may be substituted by one or more substituents, for example an aryl or cycloalkyl ring may be substituted by one or more alkyl, alkoxy, cyano, nitro, or alkoxycarbonyl groups or halogen atoms, and an alkyl, alkenyl or alkynyl group may be substituted by alkoxy, cyano or alkoxycarbonyl groups or halogen atoms.

The process of the invention may be usefully applied in the preparation of aldehydes containing cyclopropyl groups. Certain such aldehydes are useful in the synthesis of biologically-active molecules.

The following Examples illustrate the preparation of the reducing agent (Example 1) and the reduction process according to the invention (Examples 2 to 24).

Example 1 – Preparation of cadmium borohydride solution

Cadmium chloride was recrystallised from dimethylformamide; it is thought that this solid has the empirical formula $CdCl_2$.1.5 DMF.1 mol of this complex was added to a 1:1 mix of acetonitrile and dimethylformamide, and 2 mol of $NaBH_4$ were added. The resulting solution contained approximately 0.14 mmol of cadmium borohydride per millilitre of solution. The bulk solution stored well at 0 °C, with a white solid separating out slowly. This white solid has been shown not to be sodium chloride, suggesting that the cadmium borohydride reducing agent is not present as $Cd(BH_4)_2$ per se, but may be present in a complex such as $Na_2\,CdCl_2(BH_4)_2$. However this is a tentative theory which is not yet proven.

Examples 2–7

These Examples illustrate the improved yields obtained in the reduction of a range of carboxylic acid chlorides to the corresponding aldehydes using cadmium borohydride as reducing agent.

A solution of $NaBH_4$ (0.76 g) in dimethylformamide (5 ml) and acetonitrile (60 ml) was added over 10 minutes to a stirred suspension of 4.6 g (25 m mol) $CdCl_2$ in acetonitrile (25 ml) at 0 °C. The mixture was stirred for a further 10 minutes at 0 to 3 °C and then cooled to -35 °C. A solution of 20 m mol acid chloride in 20 ml acetonitrile was added over a quarter of an hour. After stirring for a further 10 minutes, the temperature was allowed to rise to room temperature and the mixture was poured onto 2N HCl (50 ml) and ice (50 g).

The aldehyde produced was isolated as its hydrazone by adding a saturated solution of 2,4-dinitrophenylhydrazine (200 ml) to the acidified solution. After warming over a steam bath, the yellow/orange precipitate of the 2,4-dinitrophenylhydrazine of the aldehyde was filtered off.

The results for various starting materials are listed in the following table, which also lists, for comparison, results obtained using sodium borohydride alone (i.e. without addition of $CdCl_2$) under the same reaction conditions. The comparison results show that in general, yields obtained using cadmium borohydride are approximately double the yields obtained using sodium borohydride.

| Example | Starting Acid Chloride | 2,4-dinitrophenylhydrazone of the Resulting Aldehyde | | |
|---|---|---|---|---|
| | | Using $Cd(BH_4)_2$ | | Using $NaBH_4$ (for comparison only) |
| | | % Yield (calculated on starting acid chloride) | M.PT. (°C) | % Yield (calculated on starting acid chloride) |
| 2 | 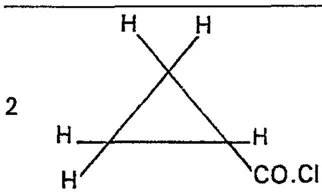 | 62 | 176–177 | 32 |
| 3 | 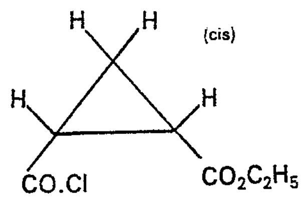 (cis) | 50 | 157–159 | 23 |
| 4 | 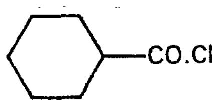 | 24 | 165–166 | 7.5 |
| 5 | 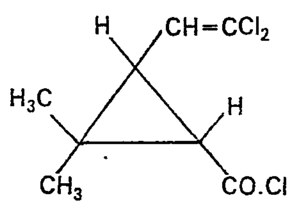 | 74 | 124-128 | 43.6 |
| 6 | 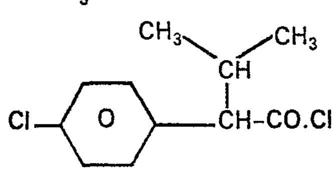 | 61 | | 25 |
| 7 | 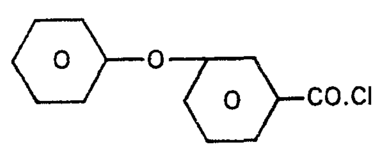 | 81 | 185–187 | 40.5 |

Examples 8-21

These Examples illustrate the yields obtained in the cadmium borohydride reduction of a range of aromatic and aliphatic acid chlorides, many containing substituents sensitive to other reducing agents.

76 mg (2 m mol) $NaBH_4$ were dissolved in 10 ml acetonitrile and 0.35 ml hexamethylphosphoramide. 370 mg cadmium chloride recrystallised from dimethylformamide were added; this amount corresponded to 1 m mol assuming a molecular formula of $CdCl_2.2.5$ DMF. The reaction mixture was cooled to −10 °C to −5 °C, and 2 m mol acid chloride in 2–3 ml acetonitrile were added rapidly and the solution stirred well for 5 minutes. Dilute HCl was added to decompose excess reducing agent. Excess 2,4-dinitrophenylhydrazine solution was added and the reaction mixture was heated on a water bath for a few minutes to complete the reaction. The precipitated 2,4-dinitrophenylhydrazone was filtered, washed and dried; in many cases, satisfactory purity was obtained without recrystallisation. The results are shown in the following table.

| Example | Starting Acid Chloride | 2,4-dinitrophenylhydrazone of the Resulting Aldehyde | | |
|---|---|---|---|---|
| | | % Yield (calculated on starting acid chloride) | M.Pt. (°C)* | Literature M.Pt. (°C)* |
| 8 | Benzoyl chloride | 76 | 241–242 | 237 (acetic acid) |
| 9 | 4-methylbenzoyl chloride | 89 | 238–240 | 233–234 |
| 10 | 4-chlorobenzoyl chloride | 74 | 272 | 270–271 (ethanol) |
| 11 | 4-nitrobenzoyl chloride | 71 | 317–318 | 320 (xylene) |
| 12 | 4-methoxybenzoyl chloride | 63 | 251–252 | 253–254 (acetic acid) |
| 13 | 4-cyanobenzoyl chloride | 67 | 307–308 (acetic acid) | 295–298 (acetic acid) |
| 14 | 2-methylbenzoyl chloride | 60 | 190–192 | 190–193 |
| 15 | 2-bromobenzoyl chloride | 62 | 199–200 | 203 (xylene) |
| 16 | 2-methoxycarbonylbenzoyl chloride | 52 | 242–243 (xylene) | – |
| 17 | trans-cinnamoyl chloride | 71 | 253–254 | 255 (acetic acid) |
| 18 | phenylacetyl chloride | 58 | 115–117 (ethanol/benzene) | 125–126 (ethanol/benzene) |
| 19 | octanoyl chloride | 56 | 103 (ethanol) | 106 |
| 20 | crotonyl chloride | 54 | 182–184 (ethyl acetate/ethanol) | 184–185 |
| 21 | pivaloyl chloride | 32 | 209–210 (ethyl acetate/ethanol) | 210 |

* The solvent used for recrystallisation of the 2,4-dinitrophenylhydrazine, if recrystallisation was performed, is shown in brackets.

Examples 22–24

Carboxylic acid bromides were reduced to the corresponding aldehydes by the following procedure. A solution of sodium borohydride (0.76 g) in dimethylformamide (5 ml) and acetonitrile (60 ml) was added over 10 minutes to a stirred suspension of cadmium chloride (4.6 g, 25 m mol) in acetonitrile (25 ml) at 0 °C. The mixture was stirred for a further 10 minutes at 0 to 3 °C and then cooled to –35 °C. A solution of the carboxylic acid bromide (20 m mol) in acetonitrile was added over a quarter of an hour. After stirring for a further 10 minutes, the temperature was allowed to rise to room temperature, and the mixture was poured onto 2N HCl (50 ml) and ice (50 g).

The aldehyde produced was isolated as its hydrazone by adding a saturated solution of 2,4-dinitrophenylhydrazine (200 ml) to the acidified solution. After warming over a steam bath, the yellow/orange precipitate of the 2,4-dinitrophenylhydrazone was filtered off.

The results obtained are given in the following table:

| Example | Starting Acid Bromide | 2,4-dinitrophenylhydrazine of the resulting aldehyde<br>% yield (based on starting acid bromide) | M.Pt. (°C) |
|---|---|---|---|
| 22 | 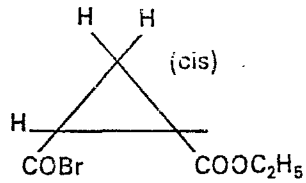 (cis) | 45 | 158–159 |
| 23 | 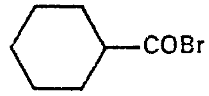 | 10 | 165–166 |
| 24 | 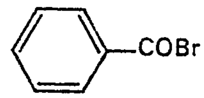 | 80 | 241–242 |

**Claims**

1. A process for the reduction of a carboxylic acid halide to the corresponding aldehyde characterised in that, the acid halide is reacted with cadmium borohydride as reducing agent.
2. A process according to claim 1 characterised in that the cadmium borohydride is present in the form of a solution in an inert polar solvent.
3. A process according to claim 2 characterised in that the cadmium borohydride solution is prepared in situ.
4. A process according to claim 2 or 3 characterised in that the solution is formed by mixing a cadmium salt with an alkali metal borohydride in an inert polar solvent.
5. A process according to claim 4 characterised in that the cadmium salt is a cadmium halide and the borohydride is sodium borohydride.
6. A process according to any one of claims 2 to 5 characterised in that the inert polar solvent is a mixture of acetonitrile with diglyme, hexamethylphosphoramide or dimethyl formamide.
7. A process according to any one of the preceding claims characterised in that the reduction is carried out at a temperature in the range -35°C to 0°C.

**Patentansprüche**

1. Verfahren zur Reduktion eines Carbonsäurehalogenids zu dem entsprechenden Aldehyd, dadurch gekennzeichnet, dass das Säurehalogenid mit Cadmiumborhydrid als Reduktionsmittel umgesetzt wird.
2. Verfahren nach Anspruch 1, dadurch gekennzeichnet, dass das Cadmiumborhydrid in Form einer Lösung in einem inerten polaren Lösungsmittel angewandt wird.
3. Verfahren nach Anspruch 2, dadurch gekennzeichnet, dass die Cadmiumborhydrid-Lösung in situ hergestellt wird.
4. Verfahren nach Anspruch 2 oder 3, dadurch gekennzeichnet, dass die Lösung hergestellt wird durch Vermischen eines Cadmiumsalzes mit einem Alkaliborhydrid in einem inerten polaren Lösungsmittel.
5. Verfahren nach Anspruch 4, dadurch gekennzeichnet, dass das Cadmiumsalz ein Cadmiumhalogenid und das Borhydrid Natriumborhydrid ist.
6. Verfahren nach einem der Ansprüche 2 bis 5, dadurch gekennzeichnet, dass das inerte polare Lösungsmittel ein Gemisch aus Acetonitril mit Diäthylenglykoldimethyläther, Hexamethylphosphoramid oder Dimethylformamid ist.
7. Verfahren nach einem der vorhergehenden Ansprüche, dadurch gekennzeichnet, dass die Reaktion bei einer Temperatur im Bereich von -35 bis 0 °C durchgeführt wird.

**Revendications**

1. Un procédé pour la réduction d'un halogénure d'acide carboxylique en l'aldéhyde correspondant, caractérisé en ce qu'on fait réagir l'halogénure d'acide avec du borohydrure de cadmium comme agent réducteur.
2. Un procédé selon la revendication 1, caractérisé en ce que le borohydrure de cadmium est présent sous la forme d'une solution dans un solvant polaire inerte.
3. Un procédé selon la revendication 2, caractérisé en ce que la solution de borohydrure de cadmium est préparée in situ.
4. Un procédé selon l'une des revendications 2 ou 3, caractérisé en ce que la solution est formée en mélangeant un sel de cadmium avec un borohydrure de métal alcalin dans un solvant polaire inerte.

5. Un procédé selon la revendication 4, caractérisé en ce que le sel de cadmium est un halogénure de cadmium et le borohydrure est du borohydrure de sodium.

6. Un procédé selon l'une quelconque des revendications 2 à 5, caractérisé en ce que le solvant polaire inerte est un mélange d'acétonitrile avec du diglyme, de l'hexaméthylphosphoramide ou du diméthylformamide.

7. Un procédé selon l'une quelconque des revendications précédentes, caractérisé en ce que la réduction est effectuée à une température comprise entre −35 °C et 0 °C.